# EUROPEAN PATENT APPLICATION

(11) **EP 4 599 833 A1**
(43) Date of publication of application: **13.08.2025**
(21) Application number: 23882740.6
(22) Date of filing: 27.10.2023
(51) Int. Cl.: A61K 33/30, A61K 33/42, A61P 1/04, A61P 29/00

(54) **PHARMACEUTICAL COMPOSITION CONTAINING ZINC POLYPHOSPHATE**

(30) Priority: 27.10.2022 JP 2022172200
(71) Applicant: Kamui Pharma, Inc., Asahikawa-shi, Hokkaido 078-8802 (JP); National University Corporation Asahikawa Medical University, Asahikawa, Hokkaido 078-8510 (JP); KNC Laboratories Co., Ltd., Kobe-shi, Hyogo 650-0047 (JP)
(72) Inventor: FUJIYA Mikihiro, Asahikawa-shi, Hokkaido 078-8510 (JP); KONISHI Hiroaki, Asahikawa-shi, Hokkaido 078-8510 (JP); OGAWA Naoki, Asahikawa-shi, Hokkaido 078-8510 (JP); IKEDA Kohei, Kobe-shi, Hyogo 650-0047 (JP); ITO Saori, Kobe-shi, Hyogo 650-0047 (JP); MIYAHARA Junichi, Kobe-shi, Hyogo 650-0047 (JP)
(74) Representative: J A Kemp LLP
(86) International application number: PCT/JP2023/038804
(87) International publication number: WO 2024/090538

(57) **Abstract**

The present invention relates to a pharmaceutical composition for treating or preventing inflammatory bowel disease comprising zinc polyphosphate as an active ingredient. The zinc polyphosphate of the present invention has intestinal barrier enhancing activity and can be used to treat or prevent inflammatory bowel disease such as ulcerative colitis and Crohn's disease.

## Description

### Technical Field

### Related application:

The present specification encompasses the contents described in the specification of Japanese Patent Application No. 2022-172200 (filed on October 27, 2022), which is the basis for the priority of the present application.

### Technical Field:

The present invention relates to a pharmaceutical composition for treating or preventing inflammatory bowel disease comprising zinc polyphosphate.

### Background Art

Although anti-inflammatory drugs are used as standard treatments for inflammatory bowel disease (IBD) such as ulcerative colitis (UC) and Crohn's disease (CD), there are no drugs that directly induce mucosal healing. While conventional anti-inflammatory drugs have been used for symptomatic treatment of IBD, the true goal of treatment of IBD is nowadays said to be "mucosal healing". Probiotics such as lactic acid bacteria are known for their high safety and certain intestinal regulation effects based on many years of dietary experience, but the mechanism of action of probiotics on the intestinal condition is still unclear in many respects. In order to elucidate this, bioactive molecules produced by probiotics are being identified and analyzed.

The present inventors have identified a long-chain polyphosphate derived from malt lactic acid bacteria as a molecule that enhances the intestinal barrier function. Then, it has been demonstrated that the long-chain polyphosphate improves decreased intestinal barrier function and intestinal injury caused by DSS treatment (see Patent Literature 1), and that it causes mucosal healing in patients with refractory ulcerative colitis (see Non Patent Literature 1). In addition, it has been reported that a Ca salt of polyphosphoric acid acts on the gastrointestinal mucosa injured in inflammatory bowel disease and exerts a specific platelet aggregation effect on the injured mucosa (Patent Literature 2).

Polyphosphate is known to have wound-healing and anti-inflammatory effects, and the development of wound dressing and dental materials using amorphous material or nanoparticles composed of calcium polyphosphate is expected (Patent Literature 3).

Polyphosphate is also known as a coagulant factor. When polyphosphate is released from platelets in blood, it activates factor XII protease in the blood, leading to a coagulation reaction. Various salts of natural polyphosphoric acid, such as Ca, Mg, Na, and K salts, have been postulated, and amorphous nanoparticles are also known to exist (Non Patent Literatures 2 and 3). Donovan *et al.* prepared nanoparticles from polyphosphate with different chain lengths and reported the size of polyphosphate and its effect on blood clotting (Non Patent Literature 4).

### Citation List

### Patent Literature

Patent Literature 1: WO 2011/125619
Patent Literature 2: WO 2021/141066
Patent Literature 3: WO 2016/079006

### Non Patent Literature

Non Patent Literature 1: Fujita et al., "Long-Chain Polyphosphate Is a Potential Agent for Inducing Mucosal Healing of the Colon in Ulcerative Colitis. " Clin Pharmacol Ther. 2019 Sep.
Non Patent Literature 2: Feng et al., "Biogenic Polyphosphate Nanoparticles from a Marine Cyanobacterium Synechococcus sp. PCC 7002: Production, Characterization, and Anti-Inflammatory Properties In Vitro. " Mar Drugs. 2018 Sep 10;16(9).
Non Patent Literature 3: Feng et al., "Biogenic Polyphosphate Nanoparticles from Synechococcus sp. PCC 7002 Exhibit Intestinal Protective Potential in Human Intestinal Epithelial Cells In Vitro and Murine Small Intestine Ex Vivo. " J Agric Food Chem. 2018 Aug 1;66(30):8026-8035.
Non Patent Literature 4: Donovan et al., "Size-controlled synthesis of granular polyphosphate nanoparticles at physiologic salt concentrations for blood clotting." Biomacromolecules. 2014 Nov 10;15(11):3976-84

### Summary of Invention

### Technical Problem

The objective of the present invention is to provide a novel pharmaceutical using a polyphosphate. For example, the objective of the present invention is to provide a pharmaceutical composition for effectively treating or preventing inflammatory bowel disease.

### Solution to Problem

The present inventors have found that among polyphosphates, zinc polyphosphate has high intestinal barrier enhancing activity. In addition, it has been found that zinc polyphosphate has excellent storage stability and is suitable for a practical pharmaceutical composition. It has been further found that zinc polyphosphate exerts an effect of suppressing the expression of inflammatory cytokines in the intestinal tract of animals having received the zinc polyphosphate.

The present invention is based on the above findings and, in the first aspect, provides the following [1] to [18].
[1] A pharmaceutical composition for treating or preventing inflammatory bowel disease comprising zinc polyphosphate as an active ingredient.
[2] The pharmaceutical composition according to [1], wherein a zeta potential of the zinc polyphosphate is -20 mV or less.
[3] The pharmaceutical composition according to [1] or [2], wherein a zinc content of the zinc polyphosphate is from 10 to 60%, preferably from 20 to 55%, and more preferably from 30 to 50%.
[4] The pharmaceutical composition according to any one of [1] to [3], wherein an average particle size of the zinc polyphosphate is from 1 to 1000 nm.
[5] The pharmaceutical composition according to any one of [1] to [4], wherein the zinc polyphosphate is obtained by reacting polyphosphate with zinc chloride under an alkaline condition.
[6] The pharmaceutical composition according to any one of [1] to [5], wherein a polyphosphate moiety of the zinc polyphosphate is linear polyphosphate.
[7] The pharmaceutical composition according to any one of [1] to [6], wherein a polyphosphate moiety of the zinc polyphosphate is obtained by an enzymatic synthesis process.
[8] The pharmaceutical composition according to any one of [1] to [7], wherein the zinc polyphosphate is hardly soluble in water.
[9] The pharmaceutical composition according to any one of [1] to [8], wherein an average chain length of a polyphosphate moiety of the zinc polyphosphate is 1.1 phosphate units or more and preferably 1.2 phosphate units or more.
[10] The pharmaceutical composition according to any one of [1] to [9], wherein an average chain length of a polyphosphate moiety of the zinc polyphosphate is less than 100 phosphate units, preferably 50 phosphate units or less, more preferably 30 phosphate units or less, and still more preferably 15 phosphate units or less.
[11] The pharmaceutical composition according to any one of [1] to [10], wherein the composition has intestinal barrier enhancing activity.
[12] The pharmaceutical composition according to any one of [1] to [11], wherein a half-life of the intestinal barrier enhancing activity of the zinc polyphosphate under a condition at 40°C and 75% humidity is 2 weeks or more.
[13] The pharmaceutical composition according to any one of [1] to [12], wherein a half-life of the intestinal barrier enhancing activity of the zinc polyphosphate under a condition at 60°C or less and 75% humidity is 2 weeks or more.
[14] The pharmaceutical composition according to any one of [1] to [13], wherein a half-life of the intestinal barrier enhancing activity of the zinc polyphosphate in an artificial gastric juice is 120 minutes or more.
[15] A pharmaceutical composition for enhancing intestinal barrier, comprising zinc polyphosphate as an active ingredient.
[16] A pharmaceutical composition comprising zinc polyphosphate as an active ingredient for inhibiting expression of one or more inflammatory cytokines selected from intestinal inflammatory cytokines such as IL1β, TNF, IL6, and IL12B.
[17] A method for predicting an intestinal barrier enhancement rate of a polyphosphoric acid metal salt, comprising:
   measuring a zeta potential of the polyphosphoric acid metal salt; and
   predicting an intestinal barrier enhancement rate of the polyphosphoric acid metal salt from a change in the zeta potential during a storage period.
[18] Use of zinc polyphosphate in the manufacture of a pharmaceutical composition for treating or preventing inflammatory bowel disease.

In the above [1] to [18], the zinc polyphosphate preferably shows signal peaks at 2θ = 30 to 36° and 56 to 62°, and more preferably, further shows a signal peak at 2θ = 4 to 7° in X-ray diffraction.

In [15] to [18] above, the zinc polyphosphate has all the characteristics specified in [2] to [16] above.

The present invention is based on the above findings and, in the second aspect, provides the following [1] to [16].
[1] A method for treating or preventing inflammatory bowel disease, comprising administering zinc polyphosphate to a subject in need thereof.
[2] The method according to [1], wherein a zeta potential of the zinc polyphosphate is -20 mV or less.
[3] The method according to [1], wherein a zinc content of the zinc polyphosphate is from 10 to 60%, preferably from 20 to 55%, and more preferably from 30 to 50%.
[4] The method according to [1], wherein an average particle size of the zinc polyphosphate is from 1 to 1000 nm.
[5] The method according to [1], wherein the zinc polyphosphate is obtained by reacting polyphosphate with zinc chloride under an alkaline condition.
[6] The method according to [1], wherein a polyphosphate moiety of the zinc polyphosphate is linear polyphosphate.
[7] The method according to claim 1, wherein a polyphosphate moiety of the zinc polyphosphate is obtained by an enzymatic synthesis process.
[8] The method according to claim 1, wherein the zinc polyphosphate is hardly soluble in water.
[9] The method according to [1], wherein an average chain length of a polyphosphate moiety of the zinc polyphosphate is 1.1 phosphate units or more and preferably 1.2 phosphate units or more.
[10] The method according to [1], wherein an average chain length of a polyphosphate moiety of the zinc polyphosphate is less than 100 phosphate units, preferably 50 phosphate units or less, more preferably 30 phosphate units or less, and still more preferably 15 phosphate units or less.
[11] The method according to [1], wherein the zinc polyphosphate has intestinal barrier enhancing activity.
[12] The method according to [1], wherein a half-life of intestinal barrier enhancing activity of the zinc polyphosphate under a condition at 40°C and 75% humidity is 2 weeks or more.
[13] The method according to [1], wherein a half-life of intestinal barrier enhancing activity of the zinc polyphosphate under a condition at 60°C or less and 75% humidity is 2 weeks or more.
[14] The method according to [1], wherein a half-life of intestinal barrier enhancing activity of the zinc polyphosphate in an artificial gastric juice is 120 minutes or more.
[15] A method for enhancing an intestinal barrier in a subject, comprising administering zinc polyphosphate to a subject in need thereof.
[16] A method for inhibiting expression of one or more inflammatory cytokines selected from intestinal inflammatory cytokines such as IL1β, TNF, IL6, and IL12B, the method comprising administering zinc polyphosphate to a subject in need thereof.

The present invention is based on the above findings and, in the third aspect, provides the following [1] to [14].
[1] A zinc polyphosphate for use in the treatment or prevention of inflammatory bowel disease.
[2] The zinc polyphosphate for use according to [1], wherein a zeta potential of the zinc polyphosphate is -20 mV or less.
[3] The zinc polyphosphate for use according to [1] or [2], wherein a zinc content of the zinc polyphosphate is from 10 to 60%, preferably from 20 to 55%, and more preferably from 30 to 50%.
[4] The zinc polyphosphate for use according to any one of [1] to [3], wherein an average particle size of the zinc polyphosphate is from 1 to 1000 nm.
[5] The zinc polyphosphate for use according to any one of [1] to [4], wherein the zinc polyphosphate is obtained by reacting polyphosphate with zinc chloride under an alkaline condition.
[6] The zinc polyphosphate for use according to any one of [1] to [7], wherein a polyphosphate moiety of the zinc polyphosphate is linear polyphosphate.
[7] The zinc polyphosphate for use according to any one of [1] to [6], wherein a polyphosphate moiety of the zinc polyphosphate is obtained by an enzymatic synthesis process.
[8] The zinc polyphosphate for use according to any one of [1] to [7], wherein the zinc polyphosphate is hardly soluble in water.
[9] The zinc polyphosphate for use according to any one of [1] to [8], wherein an average chain length of a polyphosphate moiety of the zinc polyphosphate is 1.1 phosphate units or more and preferably 1.2 phosphate units or more.
[10] The zinc polyphosphate for use according to any one of [1] to [9], wherein an average chain length of a polyphosphate moiety of the zinc polyphosphate is less than 100 phosphate units, preferably 50 phosphate units or less, more preferably 30 phosphate units or less, and still more preferably 15 phosphate units or less.
[11] The zinc polyphosphate for use according to any one of [1] to [10], wherein the zinc polyphosphate has intestinal barrier enhancing activity.
[12] The zinc polyphosphate for use according to any one of [1] to [11], wherein a half-life of the intestinal barrier enhancing activity of the zinc polyphosphate under a condition at 40°C and 75% humidity is 2 weeks or more.
[13] The zinc polyphosphate for use according to any one of [1] to [12], wherein a half-life of the intestinal barrier enhancing activity of the zinc polyphosphate under a condition at 60°C or less and 75% humidity is 2 weeks or more.
[14] The zinc polyphosphate for use according to any one of [1] to [13], wherein a half-life of the intestinal barrier enhancing activity of the zinc polyphosphate in an artificial gastric juice is 120 minutes or more.

In the above [1] to [14], the zinc polyphosphate preferably shows signal peaks at 2θ = 30 to 36° and 56 to 62°, and more preferably, further shows a signal peak at 2θ = 4 to 7° in X-ray diffraction.

### Advantageous Effects of Invention

The present invention enables new treatment and prevention of inflammatory bowel disease such as ulcerative colitis and Crohn's disease.

### Brief Description of Drawings

[Figure 1] Figure 1 shows a comparison of the activity between sodium polyphosphate and various divalent metal salts of polyphosphoric acid. The graph indicates an *ex vivo* intestinal barrier enhancement rate.
[Figure 2] Figure 2 shows a comparison of the stability among sodium polyphosphate, calcium polyphosphate, and zinc polyphosphate after storage at 60°C and 75% RH (RH: relative humidity). The graph shows a change over time in the *ex vivo* intestinal barrier enhancement rate.
[Figure 3] Figure 3 shows the correlation between the *ex vivo* activity and zeta potential of calcium polyphosphate or zinc polyphosphate. The graph shows a change over time in the *ex vivo* intestinal barrier enhancement rate.
[Figure 4] Figure 4 shows the bioactivity of sodium polyphosphate, calcium polyphosphate, or zinc polyphosphate after treatment with artificial gastric acid (pH 1). The graph shows a change over time in the *ex vivo* intestinal barrier enhancement rate.
[Figure 5] Figure 5 shows a comparison of efficacy exerted by various polyphosphates in DSS model mice. A: experimental protocol, B: intestinal length of the excised colon.
[Figure 6] Figure 6 shows the shapes of sodium polyphosphate (A), calcium polyphosphate (B), and zinc polyphosphate (C) observed by electron microscopy.
[Figure 7] Figure 7 shows the powder X-ray crystal diffraction chart of zinc polyphosphate Test 15-2.
[Figure 8] Figure 8 shows the results of examining a dose of zinc polyphosphate. A: experimental protocol, B: intestinal length of the excised colon. A: experimental protocol, B: intestinal length of the excised colon.
[Figure 9] Figure 9 shows an evaluation of efficacy of zinc polyphosphate after 8 weeks of storage under harsh conditions (at 60°C and 75% RH). A: experimental protocol, B: intestinal length of the excised colon.
[Figure 10] Figure 10 shows the intestinal barrier enhancement rate of zinc polyphosphate Test 15-12 or chain-shortened Test 15-12. From left, untreated (control), oxidative stress loaded (NH₂Cl), oxidative stress loaded + Test 15-12 treated (NH₂Cl + Test 15-12), oxidative stress loaded + chain-shortened Test 15-12 treated (NH₂Cl + Test 15-12).

### Description of Embodiments

### 1. Zinc polyphosphate

The "zinc polyphosphate" according to the present invention is a zinc salt of polyphosphoric acid. In the above-mentioned "zinc polyphosphate," it is not necessary that all hydroxyl groups of phosphate (-[PO(OH)O]-) constituting the polyphosphate form zinc salts. The zinc polyphosphate may partly contain other metal salts, such as sodium polyphosphate or calcium polyphosphate, as long as the purpose of the present invention is not impaired. In addition, the zinc polyphosphate may also contain zinc hydroxide, zinc oxide, zinc phosphate and other zinc inorganic salts, calcium phosphate and other inorganic salts, calcium carbonate and other inorganic carbon compounds, as long as the purpose of the present invention is not impaired.

### (1) Polyphosphate

The "polyphosphate", which constitutes zinc polyphosphate, is a condensed phosphate compound composed of dehydrated and condensed phosphoric acid (H₃PO₄), and may be either linear, cyclic, or branched. Preferably, the polyphosphate is linear.

As used herein, the number (n) of "phosphate units" in the linear portion of polyphosphate is used to indicate the average chain length of the polyphosphate. For example, an average chain length of 10 phosphate units (n = 10) or more means that the number of repeats of phosphate units included in the linear portion of polyphosphate is 10 or more.

The "average chain length" of polyphosphate constituting the zinc polyphosphate of the present invention is not particularly limited, and the lower limit is, for example, 1.1 phosphate units or more, preferably 1.2 phosphate units or more (e.g., 1.3, 1.4, 1.5, 1.6, 1.7, 1.8, 1.9, 2.0 phosphate units or more). The upper limit is also not particularly limited, and the average chain length is, for instance, less than 100 phosphate units, preferably 50 phosphate units or less, more preferably 30 phosphate units or less, and still more preferably 15 phosphate units or less. For example, the "average chain length" of the polyphosphate of the present invention is in a range of 1.1 phosphate units or more and less than 100 phosphate units, preferably 1.1 phosphate units or more and less than 50 phosphate units, more preferably 1.2 to 30 phosphate units, and still more preferably 1.2 to and 15 phosphate units. The zinc polyphosphate of the present invention, even when the chain is shortened, has a high intestinal barrier enhancing capacity and can effectively prevent or improve (cure or alleviate) inflammatory bowel disease.

The zinc polyphosphate of the present invention should be hardly soluble in water. Meanwhile, although the zinc polyphosphate of the present invention is preferably formed as nanoparticles, the form may be other than nanoparticles, such as a glass-like crushed material.

Polyphosphate may be chemically synthesized, synthesized *in vitro* using a biomolecule such as an enzyme, or synthesized using, for instance, polyphosphate-producing microorganisms. When linear polyphosphate is synthesized, it is preferable to synthesize it *in vitro* using a biomolecule such as an enzyme or to synthesize it using microorganisms etc., because the linear polyphosphate can be produced with high efficiency.

Examples of a method of chemically synthesizing polyphosphate include a method in which a reaction solution containing sodium phosphate as a raw material is heated for dehydration and condensation. The heating temperature may be, for example, from 150 to 350°C.

Examples of the method of synthesizing polyphosphate *in vitro* using a biomolecule such as an enzyme include a method including: using, as the "biomolecule such as an enzyme", a polyphosphate kinase (PPK), which is an enzyme that synthesizes a polyphosphate; and synthesizing polyphosphate by an action of the PPK enzyme while using ATP as a raw material. It has been reported that many probiotics such as *Lactobacillus rhamnosus* GG strain and strains belonging to *Lactobacillus brevis* contain PPK, and the gene sequences of the corresponding enzymes have been published in DB.

PPK may be acceptable if polyphosphate can be synthesized using ATP as a substrate, may be obtained from a given microorganism strain expressing PPK, or may be a commercially available one that can be purchased. The PPK may be, for example, a PPK derived from *Propionibacterium freudenreichii* subsp. *shermanii.*

The enzymatic reaction by PPK is reversible, but when a large amount of ADP compared to ATP is present in the reaction solution, the polyphosphate degradation reaction becomes dominant so that the ADP/ATP ratio reaches equilibrium. Therefore, for efficient synthesis of polyphosphate, it is desirable to keep the ADP concentration in the reaction solution low, for example, by conjugating an ATP continuous regeneration reaction system with creatine kinase or pyruvate kinase. Other conditions such as the composition of the reaction solution, reaction temperature, and reaction time may be set, if appropriate, according to, for instance, the scale of synthesis to optimize the PPK activity.

As an example, the following shows the reaction conditions for the conjugation of a continuous ATP regeneration reaction system with pyruvate kinase during polyphosphate synthesis while using PPK derived from *Propionibacterium freudenreichii* subsp. *shermanii.* Mix 680 µL of 2 mol/L Tris-HCl pH 9.0, 0.1 g of phosphoenolpyruvate, 72 mg of adenosine 5'-trisphosphate disodium trihydrate, 160 µL of 1 mol/L phosphate buffer pH 6.0, 60 µL of 2 mol/L magnesium chloride, 1 mL of 2 mol/L acetic acid buffer pH 6.0, and 2.1 mL of purified water in a 10-mL sample tube, and heat the mixture at 40°C for 30 minutes. Add, after heating, 2.5 µL of 240 U/mL polyphosphate kinase, and further heat the mixture at 40°C for 5 minutes. Then, add 2.5 µL of 1690 U/mL pyruvate kinase, set the temperature to 40°C, where the reaction time may be set to 0.5 to 36 hours. The reaction time may be set, if appropriate, according to the molecular weight and yield of the target polyphosphate, and the reaction time is preferably about 20 hours to produce, for instance, polyphosphate with a high molecular weight in high yield.

Examples of the synthesis method using microorganisms include a method including culturing, under a suitable culture condition, a polyphosphate-producing microorganism, so that the microorganism can produce polyphosphate. Examples of the polyphosphate-producing microorganism include *Lactobacillus rhamnosus* GG strain, *Lactobacillus brevis* SBC8803 strain, strains belonging to *Lactobacillus,* strains belonging to *Bifidobacterium,* strains belonging to *Enterococcus,* strains belonging to *Lactococcus,* strains belonging to *Pediococcus,* strains belonging to *Leuconostoc,* strains belonging to *Streptococcus,* strains belonging to *Bacteroides,* strains belonging to *Eubacterium,* or strains belonging to *Clostridium.*

The polyphosphate may be synthesized by culturing the microorganism in an appropriate culture medium and under appropriate culture temperature conditions that can sustain the growth of the microorganism used. The synthesized polyphosphate may be recovered from the culture medium after culturing or by crushing the microorganism after culturing.

In the step of purifying the synthesized polyphosphate, it is possible to combine and use, if appropriate, purification methods generally used in the art, such as size exclusion chromatography, ion exchange chromatography, affinity chromatography, high performance liquid chromatography (HPLC), dialysis, salt precipitation, ammonium sulfate precipitation, sedimentation, and crystallization. The purification method used may be determined, if appropriate, according to, for instance, the method used in the polyphosphate synthesis process, the desired degree of purification, and the desired yield.

Preferably, the polyphosphate used in the preparation of the zinc polyphosphate of the present invention is linear polyphosphate obtained by an enzymatic synthesis process.

### (2) Zinc polyphosphate

The "zinc content" of the zinc polyphosphate of the present invention is preferably from 10 to 60%, more preferably from 20 to 55%, and still more preferably from 30 to 50%. The "zinc content" is particularly preferably from 32.5 to 42.5% for enzymatically synthesized long-chain polyphosphate as a raw material, and is particularly preferably from 40 to 50% for chemically synthesized short-chain polyphosphate as a raw material. Zinc polyphosphate in this range can achieve high intestinal barrier enhancing activity. Note that the "zinc content" refers to the percentage (%) by weight of the element zinc in the bulk zinc polyphosphate free of excipients and so on.

The zinc polyphosphate may contain some divalent metal salts of polyphosphoric acid such as calcium or magnesium salts, or monovalent metal salts such as sodium or potassium salts. In such cases, the content of a metal(s) other than zinc is in the range of 0 to 30%, preferably 0 to 20%, more preferably 0 to 10%, and still more preferably 0 to 7%. Note that the "content of a metal(s) other than zinc" refers to the percentage (%) by weight of the element metal(s) other than zinc in the bulk zinc polyphosphate free of excipients and so on.

The zeta potential of the zinc polyphosphate of the present invention is preferably -20 mV or less, and particularly preferably -30 mV or less. As described below, a change in the zeta potential of a polyphosphoric acid divalent metal salt correlates with a change in the intestinal barrier enhancement rate of the polyphosphoric acid divalent metal salt. Therefore, the intestinal barrier enhancing activity of the zinc polyphosphate can be predicted based on the change in the zeta potential of the zinc polyphosphate during storage.

In the present invention, the zinc polyphosphate is preferably hardly soluble in water, and should therefore be highly effective in efficiently reaching the intestinal mucosa, the site of action, and being taken up by epithelial cells through endocytosis. The water-insoluble zinc polyphosphate is also advantageous in terms of manufacturing cost because the bulk drug production step, such as the recovery, washing, and drying, is simple.

The zinc polyphosphate in the present invention is preferably "nanoparticles". The term "nanoparticles" means particles with a particle size on the order of nanometers. The nanoparticles are each often like a smooth sphere with a relatively uniform particle size, and have desirable properties in terms of formulation.

Specifically, the "average particle size" of the zinc polyphosphate of the present invention is preferably from 1 nm to 1000 nm and more preferably from 10 nm to 300 nm, and fine particles may aggregate to have a particle size in this range. The average particle size can be calculated from the particle size distribution according to known techniques.

The zinc polyphosphate of the present invention should exhibit signal peaks at 2θ = 30 to 36° and 56 to 62° in X-ray diffraction. More preferably, the zinc polyphosphate additionally exhibits a signal peak at 2θ = 4 to 7° in X-ray diffraction.

The zinc polyphosphate according to the present invention is characterized in that the zinc polyphosphate retains higher "intestinal barrier enhancing activity" than other polyphosphates and is also superior in terms of storage stability. The intestine has an "intestinal barrier" function that protects the intestinal tract from the intake of pathogens and toxic substances. The "intestinal barrier enhancing activity" refers to the action of enhancing this intestinal barrier function. The "intestinal barrier enhancing activity" may be evaluated by comparing the intestinal permeability with that of the control, as demonstrated in the Examples described below. For example, in *ex vivo,* an intestine excised is filled with RPMI culture medium (FUJIFILM Wako Pure Chemical Corporation) supplemented with a test substance and is cultured; next, ³H-mannitol (Perkin-Elmer) and a monochloramine solution (prepared by mixing distilled water, NH₄Cl (FUJIFILM Wako Pure Chemical Corporation), NaOCl (FUJIFILM Wako Pure Chemical Corporation), and RPMI culture medium (FUJIFILM Wako Pure Chemical Corporation)) is added to the culture medium and oxidative stress is thus applied; the amount of ³H-mannitol leaked out of the intestinal tract is measured; and the amount of ³H-mannitol in the case without the test substance is compared therewith (intestinal barrier enhancement rate (%) below) to be able to evaluate the "intestinal barrier enhancing activity". Intestinal barrier enhancement rate (%) = {1 - (cpmsample - cpmcontrol) / (cpmNH2Cl - cpmcontrol)} × 100
cpmₛₐₘₚₗₑ: amount of ³H-mannitol under oxidative stress with polyphosphate addition
cpm_{NH2Cl}: amount of ³H-mannitol under oxidative stress (without polyphosphate addition)
cpm_{control}: normal amount of ³H-mannitol (without polyphosphate addition and without oxidative stress load)

For example, the half-life of the intestinal barrier enhancing activity of zinc polyphosphate of the present invention is 2 weeks or more, preferably 4 weeks or more, and still more preferably 6 months or more under the condition at 40°C and 75% humidity. Alternatively, the half-life of the intestinal barrier enhancing activity of zinc polyphosphate of the present invention is 2 weeks or more, preferably 4 weeks or more, and still more preferably 6 months or more under the condition at 60°C or less and 75% humidity.

The zinc polyphosphate of the present invention can maintain the above intestinal barrier enhancing activity even under an acidic condition. For example, the half-life of the intestinal barrier enhancing activity of the zinc polyphosphate of the present invention in an artificial gastric juice is 120 minutes or more and preferably 360 minutes or more.

### 2. Pharmaceutical composition containing zinc polyphosphate as active ingredient

The present invention also provides a pharmaceutical composition comprising zinc polyphosphate as an active ingredient. As described above, since zinc polyphosphate has intestinal barrier enhancing activity and storage stability, the pharmaceutical composition of the present invention is useful as a "pharmaceutical composition for prevention or treatment of inflammatory bowel disease" or "pharmaceutical composition for enhancing intestinal barrier".

"Inflammatory Bowel Disease (IBD)" is a general term for chronic or remitting/relapsing inflammatory diseases of the intestinal tract, and generally refers to two diseases: Ulcerative Colitis (UC) and Crohn's Disease (CD). Ulcerative colitis is a diffuse, nonspecific inflammation of unknown cause, in which inflammation erosions and ulcers are formed on the mucosa of the large intestine, often with recurrence and remission. Crohn's disease is a general term for chronic inflammatory diseases of unknown cause, resulting in chronic inflammation and ulceration of the mucosa of the large and small intestine.

The zinc polyphosphate of the present invention also has a high intestinal barrier function-improving activity and thus effectively treats injured gastrointestinal mucosa and induces and maintains remission of inflammatory bowel disease. The present invention also provides such a "pharmaceutical composition for inducing/maintaining remission of inflammatory bowel disease".

The pharmaceutical composition of the present invention can inhibit expression of one or more inflammatory cytokines selected from intestinal inflammatory cytokines such as IL1β, TNF, IL6, and IL12B. The present invention also provides a "pharmaceutical composition for inhibiting expression of intestinal inflammatory cytokines", and the pharmaceutical composition comprising zinc polyphosphate of the present invention as an active ingredient.

The pharmaceutical composition of the present invention may contain a pharmacologically acceptable carrier and additives together with the active ingredient, zinc polyphosphate. Examples of such a carrier and additives include, but are not limited to, excipients, binders, lubricants, solvents, disintegrants, dissolution aids, suspending agents, emulsifiers, isotonic agents, stabilizers, preservatives, antioxidants, taste masking agents, coloring agents, buffers, and flow promoters, and other common carriers and additives may be used as needed.

Specifically, excipients include organic excipients, e.g., lactose, glucose, D-mannitol and other sugars, starches, crystalline cellulose and other celluloses and inorganic excipients, e.g., calcium carbonate, kaolin.

Examples of the binders include α-starch, gelatin, gum arabic, methylcellulose, carboxymethylcellulose, sodium carboxymethylcellulose, crystalline cellulose, D-mannitol, trehalose, hydroxypropyl cellulose, hydroxypropyl methylcellulose, polyvinylpyrrolidone, and polyvinyl alcohol.

Examples of the lubricants include stearic acid, fatty acid salts, e.g., stearate, talc, and silicates.

Examples of the solvents include purified water, physiological saline, and phosphate buffer.

Examples of the disintegrants include low-substituted hydroxypropyl cellulose, chemically modified cellulose and starches.

Examples of the dissolution aids include polyethylene glycol, propylene glycol, trehalose, benzyl benzoate, ethanol, sodium carbonate, sodium citrate, sodium salicylate, and sodium acetate.

Examples of the suspending agents or emulsifiers include sodium lauryl sulfate, gum arabic, gelatin, lecithin, glycerol monostearate, polyvinyl alcohol, polyvinyl pyrrolidone, celluloses such as sodium carboxymethyl cellulose, polysorbates, and polyoxyethylene hardened castor oil.

Examples of the isotonic agents include sodium chloride, potassium chloride, sugars, glycerin, and urea.

Examples of the stabilizers include polyethylene glycol, sodium dextran sulfate, other amino acids, and magnesium carbonate, which is also an acidity regulator.

Examples of the preservatives include paraoxybenzoates, chlorobutanol, benzyl alcohol, phenethyl alcohol, dehydroacetic acid, and sorbic acid.

Examples of the antioxidants include a water-soluble antioxidant, e.g., ascorbic acid, cysteine hydrochloride, sodium bisulfite, sodium metabisulfite, sodium sulfite, a fat-soluble antioxidant, e.g., ascorbyl palmitate, butylated hydroxyanisole (BHA), butylated hydroxytoluene (BHT), lecithin, propyl gallate, α-tocopherol, and a metal chelating agent, e.g., citric acid, ethylenediaminetetraacetic acid (EDTA), sorbitol, tartaric acid, phosphoric acid.

Examples of the taste and odor masking agents include sweeteners and flavoring agents, which are commonly used in the pharmaceutical field, and examples of the coloring agents include coloring agents commonly used in the pharmaceutical field.

The pharmaceutical composition of the present invention is a pharmaceutical preparation such as tablets (including sugar-coated tablets, film-coated tablets, sublingual tablets, orally disintegrating tablets), dispersions, granules, capsules (including soft capsules and microcapsules), liquids, troches, syrups, emulsions, suspensions, injectables (e.g., subcutaneous injectables, intramuscular injectables, intraperitoneal injectables), topical preparations (e.g., nasal preparations, transdermal preparations, ointment preparations), suppository (e.g., rectal suppository, vaginal suppository), foam preparations (intrarectal preparations), pellets, intranasal preparations, and transpulmonary preparations (inhalation preparations), and can be safely administered orally or parenterally (to an oral cavity, esophagus, stomach, small intestine, large intestine, rectum, etc.). The pharmaceutical composition of the present invention preferably involves oral administration, suppository administration, or rectum administration, and oral administration is particularly preferred.

The pharmaceutical composition of the present invention may be a controlled-release preparation such as a rapid-release preparation or a sustained-release preparation. In the case of oral preparation, coating may be applied for masking, enteric solubility, or persistence, if necessary. Examples of a coating base agent used for the coating include a sugar-coating base agent, a water-soluble film-coating base agent, an enteric-soluble film-coating base agent, and a sustained-release film-coating base agent.

The pharmaceutical composition of the present invention may be administered to humans or to non-human mammals. The dose and administration method are not particularly limited and may be determined, if appropriate, according to the condition, age, etc., of the individual receiving the drug.

The dose of the pharmaceutical composition of the present invention is determined, if appropriate, according to the purpose of use, route of administration, etc. For human administration, for example, the dose per day in terms of zinc polyphosphate may be selected from ranges of 0.01 mg/kg to 4 mg/kg, preferably 0.015 mg/kg to 2 mg/kg, and more preferably 0.03 mg/kg to 1 mg/kg. Alternatively, the pharmaceutical composition may be administered in one to several daily doses ranging from 0.6 to 240 mg/day, preferably 0.9 to 120 mg/day, and more preferably 1.8 to 60 mg/day per patient.

The zinc polyphosphate of the present invention is the main body of the intestinal barrier enhancing activity and is comprised of zinc polyphosphate having excellent storage stability. Thus, the zinc polyphosphate of the present invention is expected to have higher intestinal barrier enhancing activity and better storage stability than those of natural polyphosphates, which are miscellaneous mixtures, and unstable sodium polyphosphate.

The pharmaceutical composition of the present invention may be used in combination with other drugs as long as the purpose of the invention is not impaired. Examples of the pharmaceutical optionally used in combination with the pharmaceutical composition of the present invention include drugs commonly used for the treatment of inflammatory bowel disease such as ulcerative colitis and Crohn's disease, such as 5-aminosalicylic acid (5-ASA) preparations, e.g., mesalazine, salazosulfapyridine; steroids, e.g., prednisolone, methylprednisolone; anti-TNFα preparations, e.g., infliximab, adalimumab; anti-integrin antibody preparations, e.g., ENTYVIO; a JAK inhibitor Tofacitinib; thiopurine products, e.g., mercaptopurine, azathioprine; and immunosuppressive drugs, e.g., cyclosporine, tacrolimus. The timing of administration of the pharmaceutical composition of the present invention and the concomitant drug is not limited, and they may be administered at the same time or at different times.

Since the zinc polyphosphate of the present invention can be safely taken orally, it can be used as an ingredient in, for instance, foods for specified health uses, foods for special purposes, dietary supplements, health foods, functional foods, and foods for patients.

### 3. Method for producing zinc polyphosphate

The zinc polyphosphate of the present invention is preferably obtained by reacting polyphosphate with zinc chloride under an alkaline condition. For example, the pH of the polyphosphate solution is adjusted to 7.5 or more and preferably 8 to 11, and zinc chloride is then added and the resulting zinc polyphosphate is collected. Alternatively, zinc chloride is added to a polyphosphate solution at pH 4 or more (e.g., pH 4 to 7.5), the pH is then adjusted to 7.5 or more and preferably 8 to 11, and the resulting zinc polyphosphate is then collected.

As described above, polyphosphate may be reacted with zinc chloride under an alkaline condition to produce zinc polyphosphate with high intestinal barrier enhancing activity and excellent storage stability.

### 4. Method for predicting intestinal barrier enhancement rate of polyphosphoric acid metal salt

During analysis of zinc polyphosphate, the present inventors have found that a change in the zeta potential of a polyphosphoric acid divalent metal salt correlates with a change in the intestinal barrier enhancement rate of the polyphosphoric acid divalent metal salt. The present invention also provides a method for predicting an intestinal barrier enhancement rate of a polyphosphoric acid divalent metal salt based on a change in the zeta potential of the polyphosphoric acid divalent metal salt during storage.

### 5. Method of treatment and prevention using zinc polyphosphate

The present invention provides a method for treating or preventing inflammatory bowel disease, the method comprising administering zinc polyphosphate of the present invention to a subject. The zinc polyphosphate can enhance an intestinal barrier function and inhibit one or more intestinal inflammatory cytokines (one or more selected from IL1β, TNF, IL6, and IL12B) to effectively treat or prevent inflammatory bowel disease of a subject.

### Examples

Hereinafter, the present invention is further described in detail with reference to Examples. The present invention, however, is not limited to these Examples.

### Example 1: To prepare various polyphosphates

### 1. Preparation of sodium polyphosphate (PPA-Na)

680 µL of 2 mol/L Tris-HCl pH 9.0, 0.1 g of phosphoenolpyruvate, 72 mg of adenosine 5'-trisphosphate disodium trihydrate, 160 µL of 1 mol/L phosphate buffer pH 6.0, 60 µL of 2 mol/L magnesium chloride, 1 mL of 2 mol/L acetic acid buffer pH 6.0, and 2.1 mL of purified water were mixed in a 10-mL sample tube, and the mixture was heated at 40°C for 30 minutes. After heating, 2.5 µL of 240 U/mL polyphosphate kinase was added, and the mixture was further heated at 40°C for 5 minutes. Next, 2.5 µL of 1690 U/mL pyruvate kinase was added and allowed to react at 40°C for 20 hours. The reaction solution was admixed with 1.7 mL of 2 mol/L sodium chloride, and the mixture was stirred at 10°C for 10 minutes. After stirring, the supernatant was removed, 400 µL of purified water was added to the precipitate to dissolve it, and 400 µL of 3 mol/L sodium chloride was added to cause a precipitate. The precipitate was further admixed with 400 µl of purified water for dissolution, and 400 µL of 3 mol/L sodium chloride was added to cause a precipitate. After removing the supernatant, the precipitate was lyophilized to obtain long-chain sodium polyphosphate (PPA-Na) with a chain length of 600 mer or more.

### 2. Preparation of calcium polyphosphate (PPA-Ca), zinc polyphosphate (PPA-Zn), magnesium polyphosphate (PPA-Mg), and barium polyphosphate (PPA-Ba)

Sodium polyphosphate (PPA-Na) prepared in the Section 1. was dissolved in milli-Q water at 20 mg/ml, and the pH was adjusted to 10 using aqueous sodium hydroxide; next, CaCl₂ (FUJIFILM Wako Pure Chemical Corporation; product number: 036-19731), ZnCl₂ (FUJIFILM Wako Pure Chemical Corporation; product number: 260-01021), MgCl₂ (FUJIFILM Wako Pure Chemical Corporation; product number: 136-03995), and BaCl₂/2H₂O (FUJIFILM Wako Pure Chemical Corporation; product number: 025-00172) were each added using a peristaltic pump to have a metal ion/phosphorus ratio of 0.5 while maintaining the pH 10, and the mixture was stirred for 4 hours. Each sample was added to an Amicon Ultra-15 centrifugal filter unit (MWCO; 3K, MERK Millipore), and centrifuged at 3500 rpm; flow-through was then removed, and 100% ethanol was added and the sample was centrifuged again. This process was repeated twice, and the product was dried overnight in a freeze-dryer to prepare each polyphosphate, namely calcium polyphosphate (PPA-Ca), zinc polyphosphate (PPA-Zn), magnesium polyphosphate (PPA-Mg), or barium polyphosphate (PPA-Ba).

### 3. Preparation of zinc polyphosphate Test 15-2

To 1 g of sodium polyphosphate (PPA-Na) prepared in the Section 1., 50 g of water was added to make an aqueous solution. Next, 1 mol/L aqueous sodium hydroxide was added, the pH was adjusted to 9.9, and 50 mL of 1 mol/L zinc chloride solution was added dropwise over 30 minutes. Aqueous sodium hydroxide was added as needed to maintain the pH between 9.6 and 10.2 during the dropping. After completion of the dropping, the mixture was stirred at room temperature for 4 hours, and then allowed to stand at a temperature below 10°C for 16 hours; and the precipitate was collected as a precipitate by centrifugation. The precipitate was further washed with aqueous ethanol (33 v/v% ethanol), and the precipitate was again collected by centrifugation and lyophilized to prepare zinc polyphosphate Test 15-2 as white powder.

### Example 2: To compare activity between sodium polyphosphate and divalent metal salts

### Ex vivo intestinal loop study

The intestinal tract from each C57BL/6 mouse was cut into four equal lengths, which were each ligated at the ends with silk sutures (thread number: 4-0; Alfresa Corporation), and the intestinal tract was filled with RPMI culture medium (FUJIFILM Wako Pure Chemical Corporation) containing 0.1 µg/mL of each polyphosphate (PPA-Na, PPA-Ca, PPA-Zn, PPA-Mg, or PPA-Ba). After incubation in RPMI culture medium at 37°C for 2 hours, one ligature was cut open, filled with a monochloramine solution containing 1 mCi/ml ³H-mannitol (Perkin-Elmer) at 1 µCi/mL, and ligated again, so that oxidative stress was loaded. Note that the monochloramine solution was prepared by mixing 920 µl of distilled water, 1000 µl of 40 mM NH₄Cl (FUJIFILM Wako Pure Chemical Corporation), 80 µl of 5.0% NaOCl (FUJIFILM Wako Pure Chemical Corporation), and 8000 µl of RPMI culture medium. The amount of ³H-mannitol leaked out of the intestinal tract 15 and 30 minutes after filling was measured with a liquid scintillator (Perkin Elmer; Liquid Scintillation Analyzer Tri-Carb 4910TR). In the same way, the amount of ³H-mannitol during oxidative stress load without polyphosphate addition and the amount of ³H-mannitol under the condition without polyphosphate addition or oxidative stress load (control) were measured.

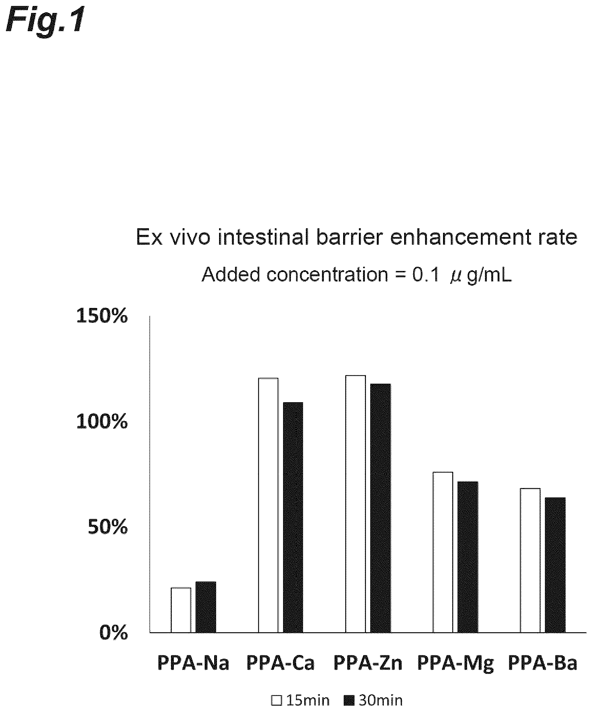

The intestinal barrier enhancement rate was calculated according to the following formula (Table 1 and Figure 1). Intestinal barrier enhancement rate (%) = {1 - (cpmsample - cpmcontrol)/(cpmNH2Cl - cpmcontrol)} × 100
cpmₛₐₘₚₗₑ: amount of ³H-mannitol under oxidative stress with polyphosphate addition
cpm_{NH2Cl}: amount of ³H-mannitol under oxidative stress (without polyphosphate addition)
cpm_{control}: normal amount of ³H-mannitol (without polyphosphate addition and without oxidative stress load)

**[Table 1]**

| | Intestinal barrier enhancement rate | |
|---|---|---|
| | 15 min. | 30 min. |
| PPA-Na | 21% | 24% |
| PPA-Ca | 120% | 109% |
| PPA-Zn | 122% | 118% |
| PPA-Mg | 76% | 71% |
| PPA-Ba | 68% | 64% |

As shown in Table 1 and Figure 1, among the various polyphosphates, zinc polyphosphate exhibited the highest intestinal barrier enhancement rate.

### Example 3: To compare stability of Na, Ca, or Zn polyphosphate under conditions at 60°C and 75% RH (Ex vivo)

### 1. Accelerated stability test

Each polyphosphate (PPA-Na, PPA-Ca, or PPA-Zn) was weighed in Eppendorf tubes at 20 mg each and stored in a small environmental tester (ESPEC CORP.; set to 60°C/75% RH) for a certain period of time in the open state.

### 2. Ex vivo intestinal loop study (60°C and 75% RH, after storage)

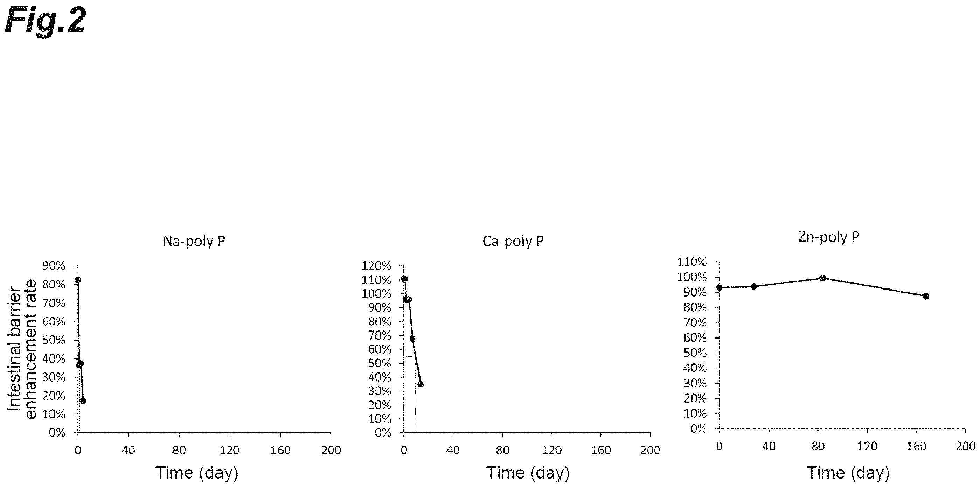

The intestinal barrier enhancement rate (%) of each polyphosphate after storage at 60°C and 75% RH was calculated according to the protocol described in Example 2 (Table 2 and Figure 2).

**[Table 2]**

| PPA-Na | | PPA-Ca | | PPA-Zn | |
|---|---|---|---|---|---|
| 60°C, 75% humidification Storage period (days) | Intestinal barrier enhancement rate | 60°C, 75% humidification Storage period (days) | Intestinal barrier enhancement rate | 60°C, 75% humidification Storage period (days) | Intestinal barrier enhancement rate |
| 0 | 82.6% | 0 | 111% | 0 | 93% |
| 1 | 36.6% | 1 | 111% | 28 | 94% |
| 2 | 37.5% | 2 | 96% | 84 | 99% |
| 4 | 17.4% | 4 | 96% | 168 | 87% |
| 7 | | 7 | 68% | | |
| 14 | | 14 | 35% | | |
| Activity half-life | 0.76 days | Activity half-life | 9.24 days | Activity half-life | 168 days or more |

As shown in Table 2 and Figure 2, zinc polyphosphate exhibited a significantly longer activity half-life than sodium polyphosphate and calcium polyphosphate.

### Example 4: Ex vivo activity and zeta potential of Ca or Zn polyphosphate

### 1. Accelerated stability test

Calcium polyphosphate (PPA-Ca) and zinc polyphosphate (PPA-Zn) were each weighed in Eppendorf tubes at 20 mg each and stored in a small environmental tester (ESPEC CORP.; set to60°C/75% RH) for a certain period of time in the open state.

### 2. Zeta potential measurement

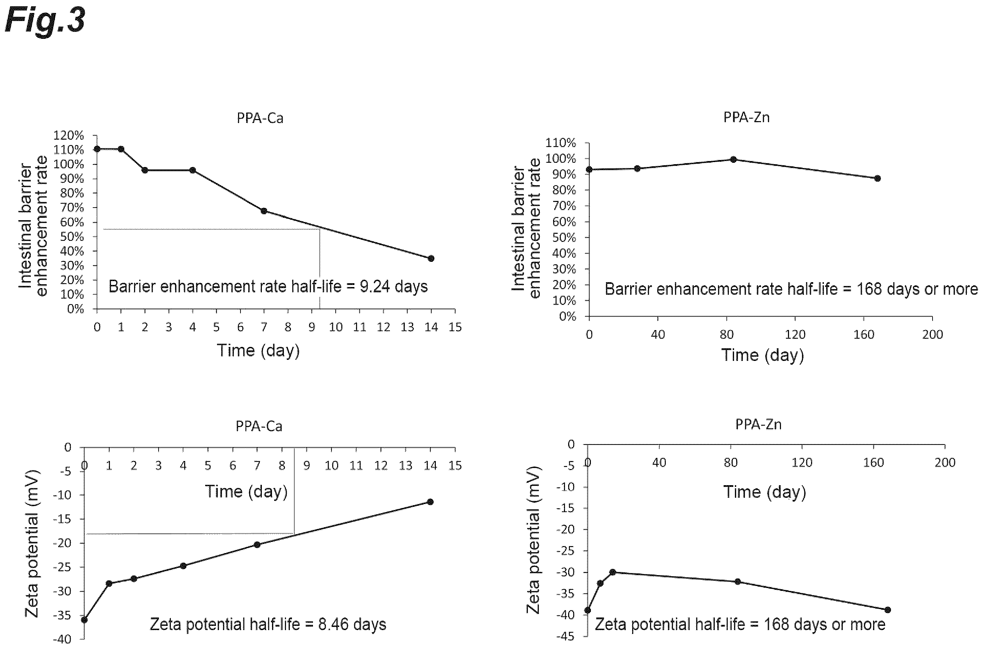

The zeta potential of calcium polyphosphate (PPA-Ca) or zinc polyphosphate (PPA-Zn) was measured using a Delsa^{™} Nano Zeta Potential and Submicron Particle Size Analyzer (Beckman Coulter, Inc) (Figure 3).

### 3. Ex vivo intestinal loop study

The intestinal barrier enhancement rate (%) of each polyphosphate after storage at 60°C and 75% RH was calculated according to the protocol described in Example 2 (Figure 3).

As shown in Figure 3, the *ex vivo* activity (intestinal barrier enhancement rate) of calcium polyphosphate or zinc polyphosphate correlates with their zeta potential.

### Example 5: Bioactivity after treatment with artificial gastric acid at pH 1 (Ex vivo test)

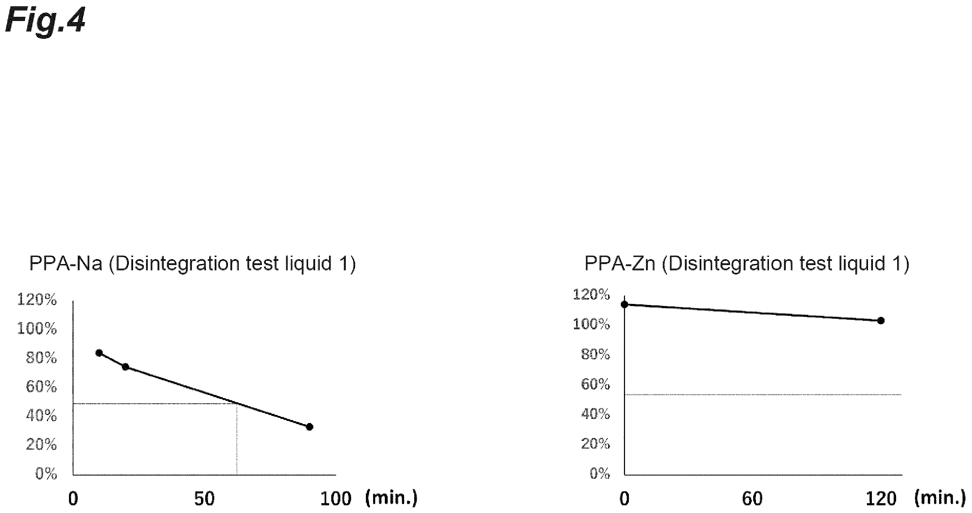

1. Drug disintegration test liquid treatment (test for stability against artificial gastric acid)
   Sodium polyphosphate (PPA-Na) or zinc polyphosphate (PPA-Zn) was treated with drug disintegration test liquid 1 (Fujifilm Wako Pure Chemical Corporation; 061-06371) for 2 hours. After treatment, they were neutralized by the addition of sodium hydroxide or Tris-HCl buffer (pH 8.0).
2. *Ex vivo* intestinal loop study (bioactivity evaluation)
   The intestinal barrier enhancement rate (%) of each polyphosphate after treatment with artificial gastric acid (pH 1) was calculated according to the protocol described in Example 2 (Figure 4).

**[Table 3]**

| PPA-Na | | | PPA-Zn | | |
|---|---|---|---|---|---|
| | Barrier enhancement rate | | | Barrier enhancement rate | |
| | 15 min | 30 min | | 15 min | 30 min |
| 10 min | 76% | 84% | 0 min | 112% | 114% |
| 20 min | 78% | 75% | 120 min | 104.3% | 103.2% |
| 90 min | 23% | 33% | | | |
| t1/2 | 56.2 min | | t1/2 | 835.7 min | |

As shown in Table 3 and Figure 4, zinc polyphosphate exhibited high *ex vivo* activity (intestinal barrier enhancement rate) and a long half-life even after treatment with artificial gastric acid (pH 1.0).

### Example 6: To compare efficacy of polyphosphate (in vivo: DSS model mouse)

### 1. DSS acute enteritis model

Dextran sodium sulfate (DSS) (MP-BioMedicals; 160110) was dissolved in distilled water to prepare 2% DSS solution. Chemical-induced enteritis was triggered in BALB/c mice (Charles River, Japan) by administering the solution as drinking water ad libitum for 5 days, and acute enteritis was developed while distilled water was supplied as drinking water ad libitum from day 6 to day 8 (necropsy day). Each polyphosphate (PPA-Na, PPA-Ca, PPA-Zn, or PPA-Mg; 5 µg/head each) or tacrolimus (TAC; 60 µg/head; Sigma CORPORATION, PHR1809) was orally administered daily from day 0 to day 7, and on day 8, the mice were subjected to various tests.

### 2. To measure intestinal length

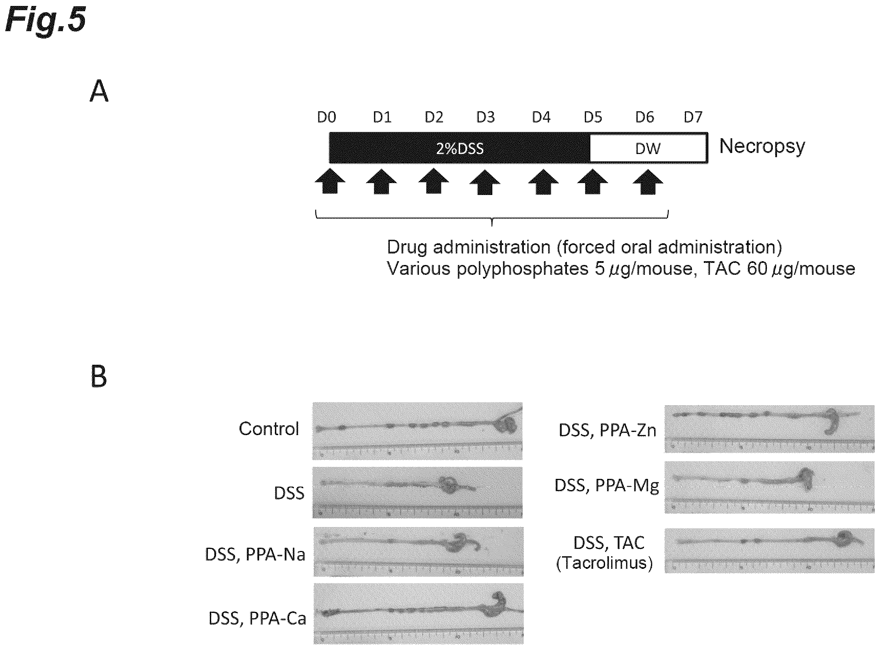

Mice were euthanized by cervical dislocation under isoflurane anesthesia, and the entire length of the colon was excised after opening the abdomen with scissors. The length of the excised colon was measured on a scale (Figure 5). Improvement rate (%) = {1 - (Average scoresample - Average scorecontrol)/(Average scoreDSS - Average scorecontrol)} × 100
Average scoreₛₐₘₚₗₑ: average length of colon in each drug group
Average score_{control}: average length of colon in control group
Average score_{DSS}: average length of colon in DSS administration group

### 3. RT-PCR

The colon was cut and open in half using scissors, and the intestinal mucosa was detached with a slide glass. Intestinal mucosal tissue was soaked in 0.5 ml TRIzol (ThermoFisher Scientific K.K.), frozen in liquid nitrogen, and stored at -80°C. After thawing each stored sample at room temperature, RNA was recovered according to the procedure instructed by ThermoFisher Scientific K.K. The recovered total RNA was purified using an RNeasy Mini kit (Qiagen) and reverse-transcribed using a High-Capacity cDNA Reverse Transcription Kit (ThermoFisher Scientific K.K.). A PCR reaction was performed, using the recovered cDNA as a template, with EagleTaq Universal Master Mix with ROX (Roche). The primers (TaqMan Gene Expression Assays; ThermoFisher Scientific K.K.) used in the reaction are as follows. IL1β: Mm01336189, TNFα: Mm00443258, IL6: Mn00446190, IFNy: Mm99999071. Changes in each gene expression level were standardized based on the expression level of Eukaryotic 18S rRNA Endogenous Control (ThermoFisher Scientific K.K.). Spectrum data was acquired using the Applied Biosystems 7300 Real Time PCR system.

### 4. Histopathological evaluation

Each colon tissue was Swiss rolled (Moolenbeek C, Lab Anim. 1981) and fixed in 10% formalin according to the procedure instructed by Moolenbeek C *et al.* The tissue was paraffin-embedded, thinly sliced to 4 µm, and stained with hematoxylin & eosin as a prepared specimen. The tissue was pathologically evaluated by inflammatory tissue scoring (Berg DJ, J Clin Invest, 1996) as described by Berg *et al.* The improvement rate (%) was calculated according to the following formula (Table 4). Improvement rate (%) = {1 - (Average scoresample - Average scorecontrol)/(Average scoreDSS - Average scorecontrol)} × 100
Average scoreₛₐₘₚₗₑ: average inflammatory tissue score in each drug group
Average score_{control}: average inflammatory tissue score in control group
Average score_{DSS}: average inflammatory tissue score in DSS administration group

**[Table 4]**

| Treatment group | Measured value | | | | | |
|---|---|---|---|---|---|---|
| | Macro findings | Micro findings | Biochemical findings (Changes* in inflammatory cytokines) *Relative value when control is set to 1 | | | |
| | Intestinal length (mm) | Histological severity | TNFα | IL1β | IL6 | IFNγ |
| Control | 125.2±4.4 | 0±0 | 1±0.6 | 1±0.7 | 1±0.7 | 1±0.4 |
| DSS | 98±7.3 | 3±0.5 | 9±10.5 | 47.1±55.3 | 68.2±166.2 | 59.8±98.9 |
| DSS, PPA-Na | 102.6±11.1 | 3.1±0.9 | 12.8±13 - | 106±106.4 | 190.4±278.2 | 148.8±255.2 - |
| DSS, PPA-Ca | 107.1±10.1 | 2.4±0.5 | 3.5±3.1 | 10±13.7 | 8.1±14.6 | 21.4±38.9 |
| DSS, PPA-Zn | 107.1±9 | 2.3±0.7 | 2.6±1.7 | 5.5±5.9 | 2.4±3.5 | 9.6±18 |
| DSS, PPA-Mg | 101.5±7.7 | 2.5±0.8 | 5.1±4.2 | 24.5±31.6 | 24.1±36.6 | 34.1±54.5 |
| DSS, TAC | 109.8±6.5 | 1.8±0.4 | 2.2±0.5 | 6.7±6.3 | 5.1±2.3 | 2±0.9 |

| Improvement rate | | | | | | |
|---|---|---|---|---|---|---|
| Drug | Macro findings | Micro findings | Biochemical findings (Changes in inflammatory cytokines) | | | |
| | Intestinal length | Histological severity | TNFα | IL1β | IL6 | IFNγ |
| - | - | - | - | - | - | - |
| - | - | - | - | - | - | - |
| PPA-Na | 17% | -5% | -47% | -128% | -182% | -151% |
| PPA-Ca | 34% * | 21% * | 69% * | 81% * | 89% | 65% |
| PPA-Zn | 34% * | 25% * | 80% * | 90% * | 98% | 85% |
| PPA-Mg | 13% | 17% | 48% | 49% | 66% | 44% |
| TAC | 43% * | 40% * | 85% * | 88% * | 94% | 98% |

As shown in Table 4 and Figure 5, zinc polyphosphate exhibited an improvement rate comparable to that of the positive control.

### Example 7: To observe shape of Na, Ca, or Zn polyphosphate by electron microscopy

### 1. Electron microscopy

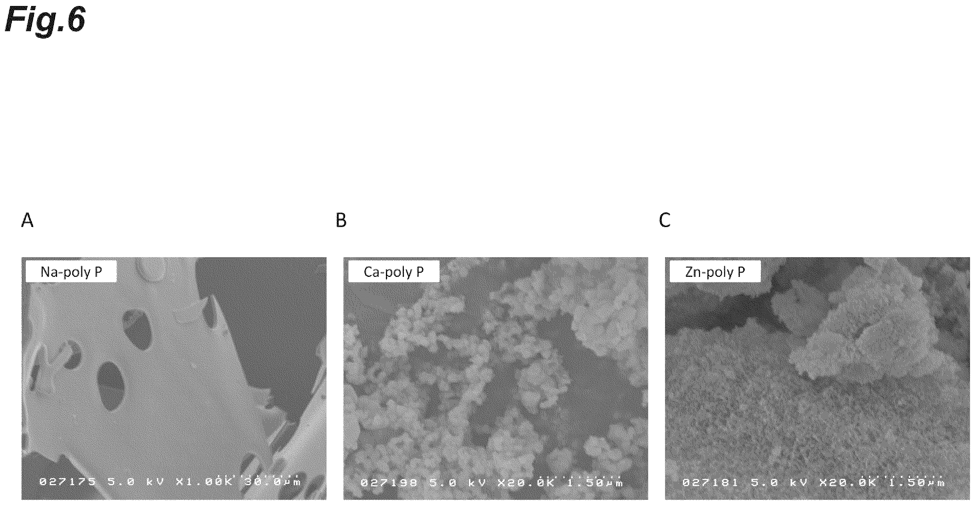

Each polyphosphate (PPA-Na, PPA-Ca, or PPA-Zn) was placed as a sample on a sample stand for electron microscopy (Okenshoji Co., Ltd, #15-1016), and the sample was coated using an ion spatter (Hitachi E1030 ion spatter). The sample was placed under a scanning electron microscope (Hitachi S-4100) and observed (Figure 6).

While sodium polyphosphate (PPA-Na) has a thin glass-like shape, calcium polyphosphate (PPA-Ca) has a typical nanoparticle shape with a relatively uniform particle size. Polyphosphate zinc (PPA-Zn) was observed to have a shape with indistinct contour, but it appeared that the fine particles had aggregated to form a large mass. The appearance of zinc polyphosphate was observed to be different depending on the recovery and drying methods, and it was considered that zinc polyphosphate could take a variety of shapes depending on the production conditions.

### 2. Characteristic Analysis

### 2.1 To check degree of polymerization of zinc polyphosphate by NMR measurement

About 10 mg of zinc polyphosphate was dissolved in about 0.7 mL of about 190 mmol/L citrate-sodium buffer (D₂O) at pH 5.5, and the analytical sample was subjected to ³¹P NMR measurement.
(i) Equipment used: Bruker Biospin 400 MHz
(ii) Analysis and analytical conditions: the signal at -5 ppm was the terminal phosphate group, the signal at -15 to -25 ppm was the internal phosphate group or the phosphate group of cyclized polyphosphate, and the total integrated value of the phosphate groups when the integrated value of the terminal phosphate group was set to 2 was calculated. Since the analyte zinc polyphosphate is prepared from linear PPA-Na, cyclic and branched polyphosphates should be rare, so that the calculated total integrated value of all the phosphate groups is considered to approximate the degree of polymerization.
(iii) Result: the degree of polymerization of Test 15-2 was calculated to be 11.3 by NMR measurement. Based on the Test 15-2 production method, the degree of polymerization of any of several zinc polyphosphate products obtained by changing detailed conditions such as the stirring time after the dropping of sodium hydroxide, and the collection, washing, and drying of a precipitate was also calculated to be in the range of 5.6 to 12.0. Conventionally, the barrier enhancing activity of polyphosphate requires long chains of 100 chains or more, and the fact that such a short chain exhibits potent activity is a feature of the present invention.

### 2.2 Powder X-ray crystal diffraction of zinc polyphosphate

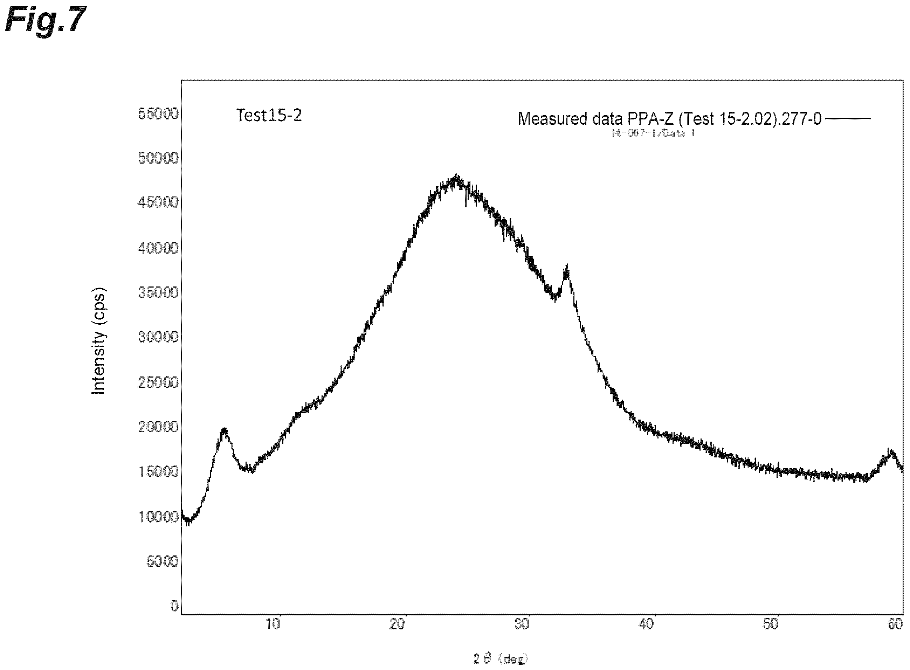

(i) Equipment used: Rigaku MiniFlexII
(ii) Analysis conditions: cathode: Cu, tube voltage: 30 kV, tube current: 15 mA, monochromatization: Ni filter, sampling width: 0.020°, scanning speed: 10°C/min, wavelength: 1.541836 Å, measurement diffraction angle range (2θ): 2 to 60°, divergence slit: 1.25°, scattering slit: 8.0 mm, light receiving slit: open.
(iii) Results: several zinc polyphosphate samples were prepared based on Test 15-2 and the Test 15-2 production method by changing detailed conditions such as the stirring time after the dropping of sodium hydroxide, and the collection, washing, and drying of a precipitate, and exhibited halo signal peaks at 2θ = 4 to 7°, 30 to 36°, and 56 to 62° (Figure 7). Conventional polyphosphates such as sodium polyphosphate and calcium polyphosphate do not exhibit such signal peaks.

### Example 8: Element analysis of each polyphosphate

20 mg of each polyphosphate (PPA-Na, PPA-Ca, PPA-Zn, or Test 15-2) was weighed on a precision electronic balance, and element analysis was performed at YAGAI-KAGAKU Co.,Ltd. (Sapporo). Moisture content was determined by the loss on drying method (drying at 105°C for 2 hours). A test liquid used was prepared by adding nitric acid and hydrogen peroxide to a sample, decomposing the sample by heat in a microwave sample pretreatment apparatus, and diluting the sample to 20 ml with distilled water. Sodium, calcium, or magnesium content was determined by a flame atomic absorption method using an atomic absorption spectrophotometer (Perkin Elmer, Analist 200), zinc content was determined by ICP mass spectrometry using an ICP mass analyzer (Agilent, Agilent 7500cx), and phosphorus content was determined by reduced molybdenum blue absorption spectrophotometry using a spectrophotometer (SHIMADZU COPORATION, UV-1800) (Table 5).

**[Table 5]**

| Analytical test items | | Element analysis | | | | | |
|---|---|---|---|---|---|---|---|
| | | Na Content | Ca Content | Mg Content | Zn Content | Phosphorus Content | Moisture content |
| Sample name | Polyphosphate | (%) | (%) | (%) | (%) | (%) | (%) |
| PPA-Na | Sodium salt | 17.8 | 0.009 | 1.89 | 0.001 | 30.6 | 5 |
| PPA-Ca | Calcium salt | 4.17 | 11.6 | 1.39 | 0.016 | 23.2 | 7.1 |
| PPA-Zn | Zinc salt | 10.6 | <0.005 | 1.19 | 17.6 | 21.2 | 8.8 |
| Test15-2 | Zinc salt | 6.27 | <0.005 | 0.737 | 36.9 | 14.9 | 7.9 |

### Example 9: To examine dose of zinc polyphosphate

### 1. DSS acute enteritis model

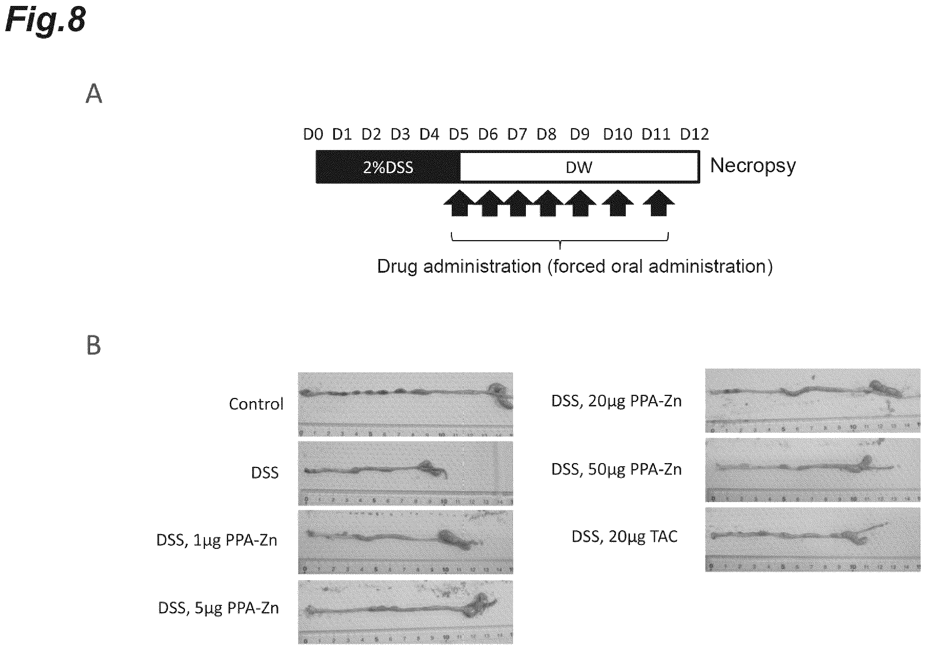

Dextran sodium sulfate (DSS) (MP-BioMedicals; 160110) was dissolved in distilled water to prepare 2% DSS solution. Chemical-induced enteritis was triggered in BALB/c mice (Charles River, Japan) by administering the solution as drinking water ad libitum for 5 days, and acute enteritis was developed while distilled water was supplied as drinking water ad libitum from day 5 to day 12 (necropsy day). Zinc polyphosphate PPA-Zn (1, 5, 20, or 50 µg/head each) or tacrolimus (20 µg/head) was orally administered daily from day 5 to day 11, and on day 12, the mice were subjected to various tests (Figure 8).

### 2. To measure intestinal length

The intestinal length after zinc polyphosphate treatment was measured according to Example 6, and the improvement rate was calculated (Figure 8).

### 3. RT-PCR analysis of mRNA expression of various cytokines

The mRNA expression levels of various cytokines (IL1β, TNFα, IL6, IFNγ, and IL17A) were analyzed according to Example 6, and the improvement rate was calculated (Table 6). The primers (TaqMan Gene Expression Assays; ThermoFisher Scientific K.K.) used in the reaction are as follows. IL1β: Mm01336189, TNFα: Mm00443258, IL6: Mn00446190, IFNy: Mm99999071, IL17A: Mm00439618.

### 4. Histopathological evaluation

Each tissue was pathologically evaluated by inflammatory tissue scoring according to Example 6, and the improvement rate was calculated (Table 6).

**[Table 6]**

| Treatment group | Measured value | | | | | | |
|---|---|---|---|---|---|---|---|
| | Macro findings | Micro findings | Biochemical findings (Changes* in inflammatory cytokines) *Relative value when control is set to 1 | | | | |
| | Intestinal length (mm) | Histological severity | TNFα | IL1β | IL6 | IFNγ | IL17A |
| Control | 132.9±9 | 0±0 | 1±0.3 | 1±0.5 | 1±0.3 | 1±0.9 | 1±0.7 |
| DSS | 96±12.6 | 3.3±0.9 | 8.2±4.3 | 124±105.5 | 98.5±214.9 | 2.4±2.3 | 157.3±262.4 |
| DSS, 1µg PPA-Zn | 109.1±14.6 | 2.9±0.8 | 4.6±3.4 | 5 8.1±80.2 | 17.1±21.3 | 1.8±2.7 | 56.5±65.1 |
| DSS, 5µ PPA-Zn | 109.3±8.7 | 2.3±0.5 | 3±1.8 | 15.7±13.5 | 15.4±21.3 | 1.7±1.9 | 52.3±57.3 |
| DSS, 20µg PPA-Zn | 115.6±8.9 | 1.5±0.5 | 2.4±1.8 | 11.1±17.6 | 3±2.4 | 1±0.6 | 14.8±21.1 |
| DSS, 50µg PPA-Zn | 109.4±18.4 | 1.9±1 | 3±4 | 54±100.7 | 31.1±71.2 | 1.1±1.9 | 32±48.1 |
| DSS, 20µg TAC | 99.4±12.6 | 2.4±1.3 | 5.4±4.5 | 85.9±117.1 | 27.2±52.4 | 1.5±0.8 | 53.9±77 |

| Improvement rate | | | | | | | |
|---|---|---|---|---|---|---|---|
| Drug | Macro findings | Micro findings | Biochemical findings (Changes in inflammatory cytokines) | | | | |
| | Intestinal length | Histological severity | TNFα | IL1β | IL6 | IFNγ | IL17A |
| - | - | - | - | - | - | - | - |
| - | - | - | - | - | - | - | - |
| 1µg PPA-Zn | 36% | 12% | 50% | 54% | 83% | 46% | 65% |
| 5µg PPA-Zn | 36% | 31% | 72% | 88% | 85% | 51% | 67% |
| 20µg PPA-Zn | 53% * | 54% | 81% | 92% | 98% | 98% | 91% |
| 50µg PPA-Zn | 36% | 42% | 72% | 57% | 69% | 90% | 80% |
| 20µg TAC | 9% | 27% | 39% | 31% | 73% | 63% | 66% |

As shown in Table 6, zinc polyphosphate in a broad range of dose exhibited an improvement rate comparable to or exceeding that of the positive control.

### Example 10: To evaluate efficacy of zinc polyphosphate Test 15-2 after long-term storage

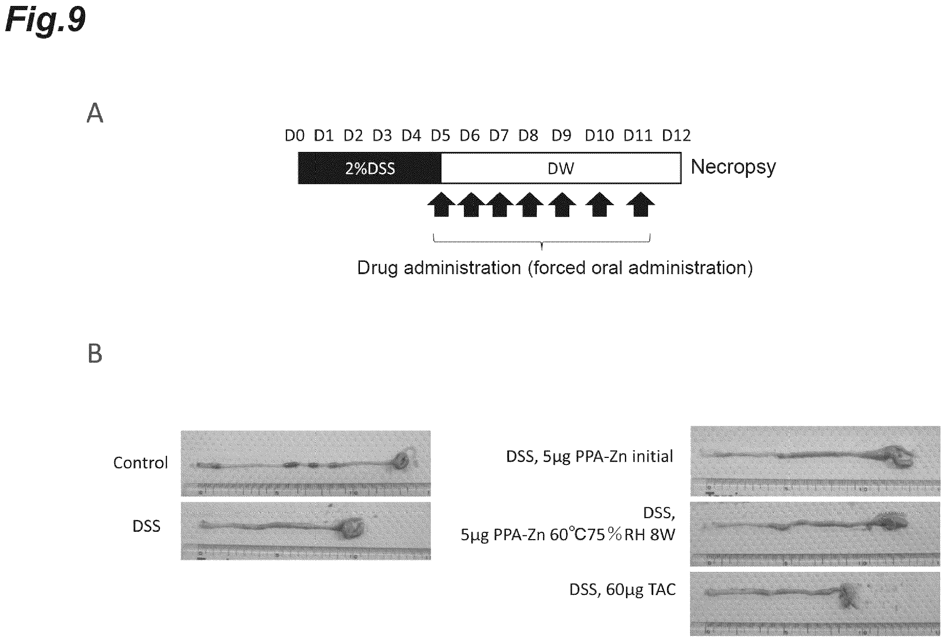

Zinc polyphosphate Test 15-2 was stored under a condition at 60°C and 75% RH for 8 weeks according to Example 2. The zinc polyphosphate Test 15-2 after storage was used to evaluate efficacy in the DSS acute enteritis model, measure the intestinal length, perform RT-PCR, and evaluate the histopathological severity according to Example 6 (Table 7, Figure 9).

### 1. DSS acute enteritis model

DDS acute enteritis model mice were generated according to Example 6 and subjected to various tests on day 12 (Figure 9).

### 2. To measure intestinal length

The intestinal length after zinc polyphosphate treatment was measured according to Example 6, and the improvement rate was calculated (Figure 9).

### 3. RT-PCR analysis of mRNA expression of various cytokines

The mRNA expression levels of various cytokines (IL1β, TNFα, IL6, IFNγ, and IL17A) were analyzed according to Example 6, and the improvement rate was calculated (Table 7). The primers (TaqMan Gene Expression Assays; ThermoFisher Scientific K.K.) used in the reaction are as follows. IL1β: Mm01336189, TNFα: Mm00443258, IL6: Mn00446190, IFNy: Mm99999071, IL17A: Mm00439618.

### 4. Histopathological evaluation

Each tissue was pathologically evaluated by inflammatory tissue scoring according to Example 6, and the improvement rate was calculated (Table 7).

**[Table 7]**

| Treatment group | Measured value | | | | | | |
|---|---|---|---|---|---|---|---|
| | Macro findings | Micro findings | Biochemical findings (Changes* in inflammatory cytokines) *Relative value when control is set to 1 | | | | |
| | Intestinal length (mm) | Histological severity | TNFα | IL1β | IL6 | IFNγ | IL17A |
| Control | 120.8±3.9 | 0±0 | 1±0.2 | 1±0.4 | 1±0.8 | 1±0.7 | 1±0.3 |
| DSS | 84.5±3.5 | 4±0 | 15.6±9.4 | 356.7±261.8 | 199.9±328.2 | 53.5±79.8 | 967.8±1258.5 |
| DSS, PPA-Zn initial | 101.1±9.6 | 2±0.8 | 6.2±4.7 | 116.1±163.5 | 36.6±52.3 | 1.5±1.7 | 231±230.3 |
| DSS, PPA-Zn 60°C, 75%RH 8W | 99.6±13.5 | 2.1±1.1 | 6.6±3.3 | 118.7±85.5 | 78.9±109 | 7±8.1 | 350.1±395 |
| DSS, TAC | 89±4.5 | 2.3±0.5 | 8.2±5 | 201.1±211.7 | 49.5±55.5 | 12.1±27.7 | 192.9±321.9 |

| Improvement rate | | | | | | | |
|---|---|---|---|---|---|---|---|
| Drug | Macro findings | Micro findings | Biochemical findings (Changes in inflammatory cytokines) | | | | |
| | Intestinal length | Histological severity | TNFα | IL1β | IL6 | IFNγ | IL17A |
| - | - | - | - | - | - | - | - |
| - | - | - | - | - | - | - | - |
| PPA-Zn initial | 46% | 50% | 65% | 68% | 82% | 99% | 76% |
| PPA-Zn 60°C, 75%RH 8W | 42% | 46% | 62% | 67% | 61% | 89% | 64% |
| TAC | 12% | 42% | 50% | 44% | 76% | 79% | 80% |

As shown in Table 7, the efficacy of zinc polyphosphate was maintained after long-term storage.

### Example 10: Effect of chain-shortened zinc polyphosphate on enhancing intestinal barrier

Zinc polyphosphate (Test 15-12; degree of polymerization = 10.8) was prepared according to Example 1. This zinc polyphosphate Test 15-12 was stored in a closed container in a thermostatic bath at 40°C for 13 days to give a chain-shortened zinc polyphosphate. When the degree of polymerization of the chain-shortened Test 15-12 was checked by ³¹P NMR measurement, and the degree of polymerization was shortened to 2.0. In addition, when X-ray diffraction of zinc polyphosphate was measured, this chain-shortened Test 15-12 still had halo peaks at 2θ = 4 to 7°, etc., in the X-ray diffraction chart. When the pH and zeta potential of the chain-shortened Test 15-12 were measured, the zeta potential = - 30.8 mV and pH = 8.3.

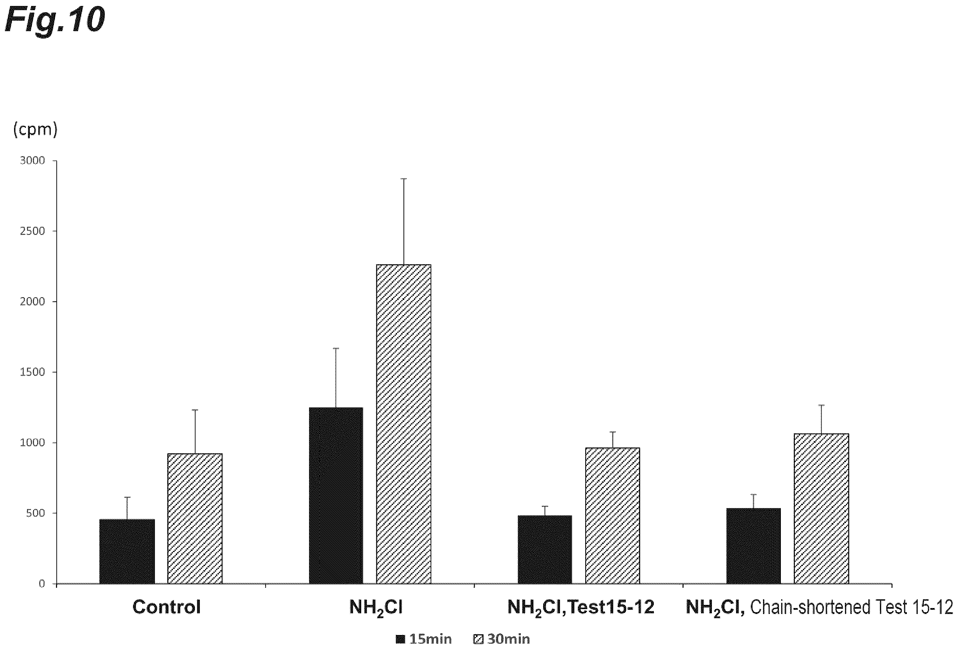

In accordance with Example 2, the intestinal barrier enhancing effects of zinc polyphosphate Test 15-12 and the chain-shortened Test 15-12 were evaluated. As shown in Figure 10, the chain-shortened Test 15-12 was added to suppress the amount of 3H-mannitol leaked out of the intestinal tract under oxidative stress load to the same level as the control, and the chain-shortened Test 15-12 thus exhibited a high intestinal barrier enhancing effect comparable to that of Test 15-12 (Figure 10). These results confirm that zinc polyphosphate has a high intestinal barrier enhancing effect even when the chain is shortened.

### Industrial Applicability

The zinc polyphosphate according to the present invention is useful for treatment and prevention of inflammatory bowel disease such as ulcerative colitis and Crohn's disease.

All the publications, patents, and patent applications cited herein are incorporated herein by reference in their entirety.

## Claims

1. A pharmaceutical composition for treating or preventing inflammatory bowel disease comprising zinc polyphosphate as an active ingredient.

2. The pharmaceutical composition according to claim 1, wherein a zeta potential of the zinc polyphosphate is -20 mV or less.

3. The pharmaceutical composition according to claim 1, wherein a zinc content of the zinc polyphosphate is from 10 to 60%.

4. The pharmaceutical composition according to claim 1, wherein an average particle size of the zinc polyphosphate is from 1 to 1000 nm.

5. The pharmaceutical composition according to claim 1, wherein the zinc polyphosphate is obtained by reacting polyphosphate with zinc chloride under an alkaline condition.

6. The pharmaceutical composition according to claim 1, wherein a polyphosphate moiety of the zinc polyphosphate is linear polyphosphate.

7. The pharmaceutical composition according to claim 1, wherein a polyphosphate moiety of the zinc polyphosphate is obtained by an enzymatic synthesis process.

8. The pharmaceutical composition according to claim 1, wherein the zinc polyphosphate is hardly soluble in water.

9. The pharmaceutical composition according to claim 1, wherein an average chain length of a polyphosphate moiety of the zinc polyphosphate is 1.1 phosphate units or more.

10. The pharmaceutical composition according to claim 1, wherein an average chain length of a polyphosphate moiety of the zinc polyphosphate is less than 100 phosphate units.

11. The pharmaceutical composition according to any one of claims 1 to 10, wherein the composition has intestinal barrier enhancing activity.

12. The pharmaceutical composition according to claim 11, wherein a half-life of the intestinal barrier enhancing activity of the zinc polyphosphate under a condition at 40°C and 75% humidity is 2 weeks or more.

13. The pharmaceutical composition according to claim 11, wherein a half-life of the intestinal barrier enhancing activity of the zinc polyphosphate under a condition at 60°C or less and 75% humidity is 2 weeks or more.

14. The pharmaceutical composition according to claim 11, wherein a half-life of the intestinal barrier enhancing activity of the zinc polyphosphate in an artificial gastric juice is 120 minutes or more.
